# EUROPEAN PATENT APPLICATION

(11) **EP 3 782 644 A1**
(43) Date of publication of application: **24.02.2021**
(21) Application number: 19204344.6
(22) Date of filing: 21.10.2019
(51) Int. Cl.: A61K 39/395, C07K 16/28, C07K 16/32, C12N 5/10, C12N 15/867, A61P 35/00, C12N 5/0783, C12N 15/62

(54) **ANTI-ERBB2 SINGLE CHAIN ANTIBODY, HUMAN ERBB2-TARGETING CHIMERIC ANTIGEN RECEPTOR, RECOMBINANT VECTOR, RECOMBINANT CELL, AND APPLICATION THEREOF**

(30) Priority: 23.08.2019 CN 201910784230
(71) Applicant: Beijing DCTY Biotech Co., Ltd., Beijing 102200 (CN); Jiao, Shunchang, Beijing 102200 (CN)
(72) Inventor: JIAO, Shunchang, CHINA, Beijing 102200 (CN); ZHANG, Rong, Beijing, Beijing 102200 (CN); YUAN, Han, Beijing, Beijing 102200 (CN)
(74) Representative: Isern Patentes y Marcas S.L.

(57) **Abstract**

The present invention provides an anti-ErbB2 single chain antibody, a human ErbB2-targeting chimeric antigen receptor, a recombinant vector, a recombinant cell, and an application thereof, and pertains to the technical field of immunobiotherapy for tumors, where the anti-ErbB2 single chain antibody has an amino acid sequence depicted in SEQ ID No.1; the human ErbB2-targeting chimeric antigen receptor has an amino acid sequence depicted in SEQ ID No.3; the recombinant vector includes a gene encoding the chimeric antigen receptor and an initial vector; the recombinant cell includes a natural killer (NK) cell and the recombinant vector. The anti-ErbB2 single-chain antibody of the present invention is capable of recognizing tumor cell specifically, thereby, avoiding the off-target effects of the CAR-NK cells, and having excellent cytotoxixitc effects to tumor cell continuously; the recombinant cell is capable of having a higher cytotoxixity to tumor cell and reducing the risk of cytokine storm.

SEQUENCE LISTING

<110>  Beijing DCTY Biotech Co., Ltd.
       JIAO, Shunchang

<120>  ANTI-ErbB2 SINGLE CHAIN ANTIBODY, HUMAN ErbB2-TARGETING CHIMERIC
       ANTIGEN RECEPTOR, RECOMBINANT VECTOR, RECOMBINANT CELL, AND
       APPLICATION THEREOF

<130>  GWP201908327

<150>  201910784230.4
<151>  2019-08-23

<160>  26

<170>  PatentIn version 3.5

<210>  1
<211>  246
<212>  PRT
<213>  artificial sequence

<220>
<223>  an anti-ErbB2 single chain antibody

<400>  1

Leu Glu Gln Ser Gly Pro Gly Ile Leu Gln Pro Ser Gln Thr Leu Ser
1               5                   10                  15

Leu Ile Cys Ser Phe Ser Gly Phe Ser Leu Ser Thr Ser Gly Met Gly
            20                  25                  30

Val Ser Trp Ile Arg Gln Pro Ser Gly Lys Gly Leu Glu Trp Leu Ala
        35                  40                  45

His Ile Tyr Trp Asp Asp Asp Lys Arg Tyr Asn Pro Ser Leu Lys Ser
    50                  55                  60

Arg Leu Thr Ile Ser Lys Asp Thr Ser Ser Lys Gln Val Phe Leu Lys
65                  70                  75                  80

Ile Thr Ser Val Asp Thr Ala Asp Thr Ala Thr Tyr Tyr Cys Ala Leu
                85                  90                  95

Phe Ala Tyr Tyr Asn Tyr Asp Gly Thr Val Asp Tyr Trp Gly Leu Gly
            100                 105                 110

Thr Ser Val Thr Val Ser Ser Gly Gly Gly Gly Ser Gly Gly Gly Gly
        115                 120                 125

Ser Gly Gly Gly Gly Ser Asp Ile Val Met Thr Gln Thr Pro Ser Ser
    130                 135                 140

Leu Ala Val Ser Val Gly Glu Lys Val Thr Met Ser Cys Lys Ser Ser
145                 150                 155                 160

Gln Ser Leu Leu His Ser Arg Thr Arg Lys Asn Tyr Leu Ala Trp Tyr
                165                 170                 175

Gln Gln Lys Leu Gly Gln Ser Pro Lys Leu Leu Ile Tyr Trp Ala Ser
            180                 185                 190

Thr Arg Glu Ser Gly Val Pro Asp Arg Phe Thr Gly Ser Gly Ser Gly
        195                 200                 205

Thr Asp Phe Thr Leu Thr Ile Ser Ser Val Glu Ala Glu Asp Leu Ala
    210                 215                 220

Val Tyr Tyr Cys Lys Gln Ser Tyr Asn Leu Tyr Thr Phe Gly Gly Gly
225                 230                 235                 240

Thr Lys Leu Glu Ile Lys
                245

<210>  2
<211>  738
<212>  DNA
<213>  artificial sequence

<220>
<223>  a gene encoding the anti-ErbB2 single chain antibody

<400>  2
ctggagcagt caggccctgg gatattgcag ccctcccaga ccctcagttt gatctgttct      60

ttctctgggt tttcactgag tacttctggt atgggtgtaa gctggattcg ccagccttca     120

ggaaagggtc tggagtggct ggcacacatt tactgggatg atgacaagcg ctataaccca     180

tccctgaaga gccggctcac aatctccaag gatacctcca gcaagcaggt tttcctcaag     240

atcaccagtg ttgacactgc agatactgcc acatactact gtgctctctt cgcctactat     300

aactacgacg ggactgtgga ctactggggt ctaggaacct cagtcaccgt ctcctcaggc     360

ggaggcggat caggtggtgg cggatctgga ggtggcggaa gcgacattgt gatgacacag     420

actccatcct ccctggctgt gtcagtagga gagaaggtca ctatgagctg caaatccagt     480

cagagtctgc tccacagtag aacccgaaag aactatttgg cttggtatca gcagaaacta     540

gggcagtctc ctaaactact gatctactgg gcatccacta gggaatctgg ggtccctgat     600

cgcttcacag gcagtggctc tgggacagat ttcactctca ccatcagcag tgtggaggct     660

gaagacctgg cagtttatta ctgcaagcaa tcttataatc tgtacacgtt cggagggggg     720

accaagctgg aaataaaa                                                   738

<210>  3
<211>  551
<212>  PRT
<213>  artificial sequence

<220>
<223>  antigen receptor

<400>  3

Leu Leu Leu Val Thr Ser Leu Leu Leu Cys Glu Leu Pro His Pro Ala
1               5                   10                  15

Phe Leu Leu Ile Pro Leu Glu Gln Ser Gly Pro Gly Ile Leu Gln Pro
            20                  25                  30

Ser Gln Thr Leu Ser Leu Ile Cys Ser Phe Ser Gly Phe Ser Leu Ser
        35                  40                  45

Thr Ser Gly Met Gly Val Ser Trp Ile Arg Gln Pro Ser Gly Lys Gly
    50                  55                  60

Leu Glu Trp Leu Ala His Ile Tyr Trp Asp Asp Asp Lys Arg Tyr Asn
65                  70                  75                  80

Pro Ser Leu Lys Ser Arg Leu Thr Ile Ser Lys Asp Thr Ser Ser Lys
                85                  90                  95

Gln Val Phe Leu Lys Ile Thr Ser Val Asp Thr Ala Asp Thr Ala Thr
            100                 105                 110

Tyr Tyr Cys Ala Leu Phe Ala Tyr Tyr Asn Tyr Asp Gly Thr Val Asp
        115                 120                 125

Tyr Trp Gly Leu Gly Thr Ser Val Thr Val Ser Ser Gly Gly Gly Gly
    130                 135                 140

Ser Gly Gly Gly Gly Ser Gly Gly Gly Gly Ser Asp Ile Val Met Thr
145                 150                 155                 160

Gln Thr Pro Ser Ser Leu Ala Val Ser Val Gly Glu Lys Val Thr Met
                165                 170                 175

Ser Cys Lys Ser Ser Gln Ser Leu Leu His Ser Arg Thr Arg Lys Asn
            180                 185                 190

Tyr Leu Ala Trp Tyr Gln Gln Lys Leu Gly Gln Ser Pro Lys Leu Leu
        195                 200                 205

Ile Tyr Trp Ala Ser Thr Arg Glu Ser Gly Val Pro Asp Arg Phe Thr
    210                 215                 220

Gly Ser Gly Ser Gly Thr Asp Phe Thr Leu Thr Ile Ser Ser Val Glu
225                 230                 235                 240

Ala Glu Asp Leu Ala Val Tyr Tyr Cys Lys Gln Ser Tyr Asn Leu Tyr
                245                 250                 255

Thr Phe Gly Gly Gly Thr Lys Leu Glu Ile Lys Ala Thr Thr Thr Ala
            260                 265                 270

Thr Ala Thr Thr Thr Gly Gly Gly Cys Ala Cys Cys Ala Cys Thr Thr
        275                 280                 285

Gly Cys Ala Gly Gly Gly Ala Cys Gly Thr Gly Cys Gly Gly Cys Gly
    290                 295                 300

Thr Ala Cys Thr Gly Cys Thr Thr Cys Thr Gly Thr Cys Thr Cys Thr
305                 310                 315                 320

Cys Gly Thr Gly Ala Thr Thr Ala Cys Thr Cys Thr Thr Thr Ala Thr
                325                 330                 335

Thr Gly Thr Ala Ala Thr Cys Ala Thr Ala Gly Gly Ala Ala Cys Arg
            340                 345                 350

Ser Lys Arg Ser Arg Leu Leu His Ser Asp Tyr Met Asn Met Thr Pro
        355                 360                 365

Arg Arg Pro Gly Pro Thr Arg Lys His Tyr Gln Pro Tyr Ala Pro Pro
    370                 375                 380

Arg Asp Phe Ala Ala Tyr Arg Ser Arg Phe Ser Val Val Lys Arg Gly
385                 390                 395                 400

Arg Lys Lys Leu Leu Tyr Ile Phe Lys Gln Pro Phe Met Arg Pro Val
                405                 410                 415

Gln Thr Thr Gln Glu Glu Asp Gly Cys Ser Cys Arg Phe Pro Glu Glu
            420                 425                 430

Glu Glu Gly Gly Cys Glu Leu Arg Val Lys Phe Ser Arg Ser Ala Asp
        435                 440                 445

Ala Pro Ala Tyr Gln Gln Gly Gln Asn Gln Leu Tyr Asn Glu Leu Asn
    450                 455                 460

Leu Gly Arg Arg Glu Glu Tyr Asp Val Leu Asp Lys Arg Arg Gly Arg
465                 470                 475                 480

Asp Pro Glu Met Gly Gly Lys Pro Arg Arg Lys Asn Pro Gln Glu Gly
                485                 490                 495

Leu Tyr Asn Glu Leu Gln Lys Asp Lys Met Ala Glu Ala Tyr Ser Glu
            500                 505                 510

Ile Gly Met Lys Gly Glu Arg Arg Arg Gly Lys Gly His Asp Gly Leu
        515                 520                 525

Tyr Gln Gly Leu Ser Thr Ala Thr Lys Asp Thr Tyr Asp Ala Leu His
    530                 535                 540

Met Gln Ala Leu Pro Pro Arg
545                 550

<210>  4
<211>  1650
<212>  DNA
<213>  artificial sequence

<220>
<223>  a gene encoding the chimeric antigen receptor

<400>  4
cttctcctgg tgacaagcct tctgctctgt gagttaccac acccagcatt cctcctgatc      60

ccactggagc agtcaggccc tgggatattg cagccctccc agaccctcag tttgatctgt     120

tctttctctg ggttttcact gagtacttct ggtatgggtg taagctggat tcgccagcct     180

tcaggaaagg gtctggagtg gctggcacac atttactggg atgatgacaa gcgctataac     240

ccatccctga agagccggct cacaatctcc aaggatacct ccagcaagca ggttttcctc     300

aagatcacca gtgttgacac tgcagatact gccacatact actgtgctct cttcgcctac     360

tataactacg acgggactgt ggactactgg ggtctaggaa cctcagtcac cgtctcctca     420

ggcggaggcg gatcaggtgg tggcggatct ggaggtggcg gaagcgacat tgtgatgaca     480

cagactccat cctccctggc tgtgtcagta ggagagaagg tcactatgag ctgcaaatcc     540

agtcagagtc tgctccacag tagaacccga aagaactatt tggcttggta tcagcagaaa     600

ctagggcagt ctcctaaact actgatctac tgggcatcca ctagggaatc tggggtccct     660

gatcgcttca caggcagtgg ctctgggaca gatttcactc tcaccatcag cagtgtggag     720

gctgaagacc tggcagttta ttactgcaag caatcttata atctgtacac gttcggaggg     780

gggaccaagc tggaaataaa attcgtgcca gtattcctgc ccgcgaagcc cactactact     840

cccgccccta ggccacctac accggccccc acaattgcat cacaaccact cagcctccgg 900

cccgaagctt gccgccccgc tgctgggggc gccgtgcaca ctcggggcct ggactttgcc 960

tgcgatattt atatttgggc accacttgca gggacgtgcg gcgtactgct tctgtctctc 1020

gtgattactc tttattgtaa tcataggaac cgatctaagc ggtccagact gctccacagt 1080

gattacatga acatgacccc acgcaggcct gggccaacac gaaaacacta tcagccttac 1140

gcacctcccc gcgactttgc tgcctaccgc tctagattca gcgtggtgaa gcgaggaaga 1200

aaaaaactcc tttatatatt caagcagcct ttcatgcgac ctgtccagac aactcaggaa 1260

gaagacggct gttcttgtag gttcccagaa gaggaagagg gaggctgcga gctgcgcgtc 1320

aaattctccc gatcagcaga tgcacccgca taccaacagg gacagaacca gctgtacaac 1380

gaactgaatc ttggaagaag ggaggagtac gatgtgcttg ataaaaggcg cggccgggac 1440

cccgaaatgg gcggtaagcc tagacgaaaa aatcctcaag agggcctgta taatgagctt 1500

cagaaagaca agatggctga ggcgtactct gaaataggga tgaaaggaga aagaaggcgg 1560

ggaaaaggcc atgatggcct gtaccagggc ctcagcaccg ctaccaagga cacatacgat 1620

gcccttcata tgcaggccct ccccccacgg 1650

<210> 5
<211> 112
<212> PRT
<213> artificial sequence

<220>
<223> the light chain variable region

<400> 5

Asp Ile Val Met Thr Gln Thr Pro Ser Ser Leu Ala Val Ser Val Gly
1               5                   10                  15

Glu Lys Val Thr Met Ser Cys Lys Ser Ser Gln Ser Leu Leu His Ser
            20                  25                  30

Arg Thr Arg Lys Asn Tyr Leu Ala Trp Tyr Gln Gln Lys Leu Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Glu Ala Glu Asp Leu Ala Val Tyr Tyr Cys Lys Gln
                85                  90                  95

Ser Tyr Asn Leu Tyr Thr Phe Gly Gly Gly Thr Lys Leu Glu Ile Lys
            100                 105                 110

<210>  6
<211>  119
<212>  PRT
<213>  artificial sequence

<220>
<223>  the heavy chain variable region

<400>  6

Leu Glu Gln Ser Gly Pro Gly Ile Leu Gln Pro Ser Gln Thr Leu Ser
1               5                   10                  15

Leu Ile Cys Ser Phe Ser Gly Phe Ser Leu Ser Thr Ser Gly Met Gly
            20                  25                  30

Val Ser Trp Ile Arg Gln Pro Ser Gly Lys Gly Leu Glu Trp Leu Ala
        35                  40                  45

His Ile Tyr Trp Asp Asp Asp Lys Arg Tyr Asn Pro Ser Leu Lys Ser
    50                  55                  60

Arg Leu Thr Ile Ser Lys Asp Thr Ser Ser Lys Gln Val Phe Leu Lys
65                  70                  75                  80

Ile Thr Ser Val Asp Thr Ala Asp Thr Ala Thr Tyr Tyr Cys Ala Leu
                85                  90                  95

Phe Ala Tyr Tyr Asn Tyr Asp Gly Thr Val Asp Tyr Trp Gly Leu Gly
            100                 105                 110

Thr Ser Val Thr Val Ser Ser
        115

<210>  7
<211>  15
<212>  PRT
<213>  artificial sequence

<220>
<223>  the linker sequence

<400>  7

Gly Gly Gly Gly Ser Gly Gly Gly Gly Ser Gly Gly Gly Gly Ser
1               5                   10                  15

<210>  8
<211>  336
<212>  DNA

<213>  artificial sequence

<220>
<223>  the gene encoding the light chain variable region

<400>  8
gacattgtga tgacacagac tccatcctcc ctggctgtgt cagtaggaga gaaggtcact      60

atgagctgca aatccagtca gagtctgctc cacagtagaa cccgaaagaa ctatttggct     120

tggtatcagc agaaactagg gcagtctcct aaactactga tctactgggc atccactagg     180

gaatctgggg tccctgatcg cttcacaggc agtggctctg ggacagattt cactctcacc     240

atcagcagtg tggaggctga agacctggca gtttattact gcaagcaatc ttataatctg     300

tacacgttcg gaggggggac caagctggaa ataaaa                               336


<210>  9
<211>  357
<212>  DNA
<213>  artificial sequence

<220>
<223>  the gene encoding the heavy chain variable region

<400>  9
ctggagcagt caggccctgg gatattgcag ccctcccaga ccctcagttt gatctgttct      60

ttctctgggt tttcactgag tacttctggt atgggtgtaa gctggattcg ccagccttca     120

ggaaagggtc tggagtggct ggcacacatt tactgggatg atgacaagcg ctataaccca     180

tccctgaaga gccggctcac aatctccaag gatacctcca gcaagcaggt tttcctcaag     240

atcaccagtg ttgacactgc agatactgcc acatactact gtgctctctt cgcctactat     300

aactacgacg ggactgtgga ctactggggt ctaggaacct cagtcaccgt ctcctca        357


<210>  10
<211>  45
<212>  DNA
<213>  artificial sequence

<220>
<223>  the gene encoding the linker sequence

<400>  10
ggcggaggcg gatcaggtgg tggcggatct ggaggtggcg gaagc                      45


<210>  11
<211>  20
<212>  PRT
<213>  artificial sequence

<220>
<223>  the GM-CSF signal peptide domain

<400>  11

Gly Ser Gly Gly Met Leu Gly Val Val Thr His Arg Ala Glu Gly Leu
1               5                   10                  15

Ser Pro Gly Glu
            20

<210>  12
<211>  82
<212>  PRT
<213>  artificial sequence

<220>
<223>  the CD8 transmembrane domain

<400>  12

Val Pro Met Ile Thr Ile Lys Ser Asn His Glu Arg Gln Lys Gln Tyr
1               5                   10                  15

Ala Ala Arg Pro Cys Lys Trp Cys Pro Asn Ile Asn Ile Ala Gly Lys
            20                  25                  30

Val Gln Ala Pro Ser Val His Gly Ala Pro Ser Ser Gly Ala Ala Ser
        35                  40                  45

Phe Gly Pro Glu Ala Glu Trp Leu Cys Asn Cys Gly Gly Arg Cys Arg
    50                  55                  60

Trp Pro Arg Gly Gly Ser Ser Ser Gly Leu Arg Gly Gln Glu Tyr Trp
65                  70                  75                  80

His Glu

<210>  13
<211>  41
<212>  PRT
<213>  artificial sequence

<220>
<223>  the CD28 intracellular domain

<400>  13

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  14
<211>  47
<212>  PRT
<213>  artificial sequence

<220>
<223>  the 4-1BB intracellular domain

<400>  14

Arg Phe Ser Val Val Lys Arg Gly Arg Lys Lys Leu Leu Tyr Ile Phe
1               5                   10                  15

Lys Gln Pro Phe Met Arg Pro Val Gln Thr Thr Gln Glu Glu Asp Gly
            20                  25                  30

Cys Ser Cys Arg Phe Pro Glu Glu Glu Glu Gly Gly Cys Glu Leu
        35                  40                  45

<210>  15
<211>  112
<212>  PRT
<213>  artificial sequence

<220>
<223>  intracellular domain

<400>  15

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  16
<211>  63

<212>  DNA
<213>  artificial sequence

<220>
<223>  GM-CSF signal peptide   artificial sequence

<400>  16
cttctcctgg tgacaagcct tctgctctgt gagttaccac acccagcatt cctcctgatc      60

cca                                                                    63


<210>  17
<211>  249
<212>  DNA
<213>  artificial sequence

<220>
<223>  CD8 transmembrane domain

<400>  17
ttcgtgccag tattcctgcc cgcgaagccc actactactc ccgcccctag gccacctaca      60

ccggccccca caattgcatc acaaccactc agcctccggc ccgaagcttg ccgccccgct     120

gctgggggcg ccgtgcacac tcggggcctg gactttgcct gcgatattta tatttgggca     180

ccacttgcag ggacgtgcgg cgtactgctt ctgtctctcg tgattactct ttattgtaat     240

cataggaac                                                             249


<210>  18
<211>  123
<212>  DNA
<213>  artificial sequence

<220>
<223>  CD28 intracellular domain

<400>  18
cgatctaagc ggtccagact gctccacagt gattacatga acatgacccc acgcaggcct      60

gggccaacac gaaaacacta tcagccttac gcacctcccc gcgactttgc tgcctaccgc     120

tct                                                                   123


<210>  19
<211>  141
<212>  DNA
<213>  artificial sequence

<220>
<223>  4-1BB intracellular domain

<400>  19
agattcagcg tggtgaagcg aggaagaaaa aaactccttt atatattcaa gcagcctttc      60

atgcgacctg tccagacaac tcaggaagaa gacggctgtt cttgtaggtt cccagaagag     120

gaagagggag gctgcgagct g                                               141

<210>  20
<211>  336
<212>  DNA
<213>  artificial sequence

<220>
<223>  intracellular domain

<400>  20
cgcgtcaaat tctcccgatc agcagatgca cccgcatacc aacagggaca gaaccagctg      60

tacaacgaac tgaatcttgg aagaagggag gagtacgatg tgcttgataa aaggcgcggc     120

cgggaccccg aaatgggcgg taagcctaga cgaaaaaatc ctcaagaggg cctgtataat     180

gagcttcaga aagacaagat ggctgaggcg tactctgaaa tagggatgaa aggagaaaga     240

aggcggggaa aaggccatga tggcctgtac cagggcctca gcaccgctac caaggacaca     300

tacgatgccc ttcatatgca ggccctcccc ccacgg                               336


<210>  21
<211>  564
<212>  DNA
<213>  artificial sequence

<220>
<223>  ErbB2-Domain4 gene

<400>  21
atggagctgg cggccttgtg ccgctggggg ctcctcctcg ccctcttgcc ccccggagcc      60

gcgagcacct gccaccagct gtgcgcccga gggcactgct ggggtccagg gcccacccag     120

tgtgtcaact gcagccagtt ccttcggggc caggagtgcg tggaggaatg ccgagtactg     180

caggggctcc ccagggagta tgtgaatgcc aggcactgtt tgccgtgcca ccctgagtgt     240

cagccccaga atggctcagt gacctgtttt ggaccggagg ctgaccagtg tgtggcctgt     300

gcccactata aggaccctcc cttctgcgtg gcccgctgcc ccagcggtgt gaaacctgac     360

ctctcctaca tgcccatctg gaagtttcca gatgaggagg gcgcatgcca gccttgcccc     420

atcaactgca cccactcctg tgtggacctg gatgacaagg gctgccccgc cgagcagaga     480

gccagccctc tgacgtccat catctctgcg gtggttggca ttctgctggt cgtggtcttg     540

ggggtggtct ttgggatcct catc                                            564


<210>  22
<211>  188
<212>  PRT
<213>  artificial sequence

<220>
<223>  ErbB2-Domain4 protein

<400>  22

Met Glu Leu Ala Ala Leu Cys Arg Trp Gly Leu Leu Leu Ala Leu Leu
1               5                   10                  15

Pro Pro Gly Ala Ala Ser Thr Cys His Gln Leu Cys Ala Arg Gly His
            20                  25                  30

Cys Trp Gly Pro Gly Pro Thr Gln Cys Val Asn Cys Ser Gln Phe Leu
        35                  40                  45

Arg Gly Gln Glu Cys Val Glu Glu Cys Arg Val Leu Gln Gly Leu Pro
    50                  55                  60

Arg Glu Tyr Val Asn Ala Arg His Cys Leu Pro Cys His Pro Glu Cys
65                  70                  75                  80

Gln Pro Gln Asn Gly Ser Val Thr Cys Phe Gly Pro Glu Ala Asp Gln
                85                  90                  95

Cys Val Ala Cys Ala His Tyr Lys Asp Pro Pro Phe Cys Val Ala Arg
            100                 105                 110

Cys Pro Ser Gly Val Lys Pro Asp Leu Ser Tyr Met Pro Ile Trp Lys
        115                 120                 125

Phe Pro Asp Glu Glu Gly Ala Cys Gln Pro Cys Pro Ile Asn Cys Thr
    130                 135                 140

His Ser Cys Val Asp Leu Asp Asp Lys Gly Cys Pro Ala Glu Gln Arg
145                 150                 155                 160

Ala Ser Pro Leu Thr Ser Ile Ile Ser Ala Val Val Gly Ile Leu Leu
                165                 170                 175

Val Val Val Leu Gly Val Val Phe Gly Ile Leu Ile
            180                 185

<210>  23
<211>  36
<212>  DNA
<213>  artificial sequence

<220>
<223>  Reverse primer

<400>  23
ggaagatcta tagacagatg ggggtgtcgt tttggc                                36

<210>  24
<211>  35
<212>  DNA

<213>  artificial sequence

<220>
<223>  forward primer

<400>  24
cttccggaat tcgaggtcaa gctgcagcag tcagg                                35


<210>  25
<211>  30
<212>  DNA
<213>  artificial sequence

<220>
<223>  Reverse primer

<400>  25
ggtgcatgcg gatacagttg gtgcagcatc                                      30


<210>  26
<211>  32
<212>  DNA
<213>  artificial sequence

<220>
<223>  Forward primer

<400>  26
gggagctcga cattgtgctg acacaatctc ct                                   32

## Description

### TECHNICAL FIELD

The present invention relates to the technical field of immunobiotherapy for tumors, and in particular to an anti-ErbB2 single chain antibody, a human ErbB2-targeting chimeric antigen receptor, a recombinant vector, a recombinant cell, and an application thereof.

### BACKGROUND

Conventional therapeutic approaches of tumors mainly include operation, radiotherapy, chemotherapy, and stem cell transplantation developed in recent years, but they tend to be palliative. With the scientific development, tumor immunotherapy achieves a huge breakthrough in recent years, which mainly includes immune checkpoint inhibitors (e.g., anti-PDl monoclonal antibody) and chimeric antigen receptor modified T cell therapy (CAR-T). In recent years, CAR-T technology has made encouraging progress in clinical trials of the treatment of hematologic diseases and was rated as the first rank of top ten scientific breakthroughs of the year 2013 in *Science.* Since Carl June's team of the University of Pennsylvania reported the first case of targeted CD19 CAR-T therapy in the treatment of a child (Emily Whitehead) with acute B-lineage leukemia and achieved complete remission in 2013, CAR-T cellular immunotherapy has developed rapidly within a couple of years. In the meantime, expression of chimeric antigen receptor in NK cells, in particular engineered NK cells, solves a problem about therapeutic T cells having difficulties in mass production and has a good tumor-killing effect. CAR-NK cell therapy is a cellular immunotherapy with the highest targeting property, the optimum consistency and the best efficacy.

CAR-NK cells are a group of NK cells that recognize specific antigens on the NK cell surface and transmit signals by means of genetic engineering. In general, CAR-NK cells recognize specific molecules on the tumor cell through a surface chimeric antigen receptor (CAR) in an antigen-antibody or ligand-receptor recognition mode, and then activate, proliferate and play a role in killing cells through their intracellular signal transduction. NK cells modified by CAR can specifically recognize tumor-associated antigens, enabling higher targeting property, killing activity, and persistence in effector NK cells than in commonly used immune cells, overcoming the local immunosuppressive microenvironment of the tumor, and breaking through the immune tolerance state of a host.

The modified NK cell expresses such type of CAR molecule: extracellular domain generally includes a CD8α or GMCSFRα signal peptide, an antigen-recognition region or an antigen-binding domain, including a single chain variable region composed of heavy and light chain variable regions; intracellular domain is a chimera of extracellular fragments of various signaling molecules, including CD28, 4-1BB, OX-40, and CD3zeta; transmembrane region is from other molecules, including PD1, CD8, CD4, CD28, and CD3zeta(CD3ζ). So far, in the CAR-T field, a design idea of combined application of CD28 and 4-1BB intracellular domains is known as third-generation CAR-T, which can trigger a target tumor cell-killing effect well. As for affinity of antigen-recognition domain, if ScFv antibody fragments are used, their affinity does not mean the higher the better. Because Kd values of affinity of naive NK cell receptors range from around 10⁻⁴ to 10⁻⁶ M, Drent et al. found that reduced affinity of CAR domain of CD38 target to target protein helped improve cell proliferation, increase the capability of killing specific cells, and relatively reduce damage to normal tissues (Drent E, Themeli M, Poels R, de Jong-Korlaar R, Yuan H, de Bruijn J, et al. A Rational Strategy for Reducing On-Target Off-Tumor Effects of CD38-Chimeric Antigen Receptors by Affinity Optimization. Molecular therapy: the journal of the American Society of Gene Therapy. 2017;25(8):1946-58.). In addition, other scholars (Liu X, Jiang S, Fang C, Yang S, Olalere D, Pequignot EC, et al. Affinity-Tuned ErbB2 or EGFR Chimeric Antigen Receptor T Cells Exhibit an Increased Therapeutic Index against Tumors in Mice. Cancer research. 2015;75(17):3596-607.) found that the CAR domain composed of antibody with an affinity of > 10⁻⁷ M could not confer cell activation at a higher level. Therefore, regulation of the affinity of CAR domain will help improve the killing potential, proliferation efficiency and safety of CAR-NK cells.

ErbB2 (also known as ErbB2, NEU, or CD340) is a 185 kDa cell membrane receptor encoded by proto-oncogene erbB-2, which is a member of the epidermal growth factor receptor (EGFR) family. In highly ErbB2-expressed tumor cells, both Ras-MAPK and PI3K-Akt feature high signaling activity, strong cell proliferation ability, suppressed mechanisms of differentiation, maturation and apoptosis, and high degree of cellular malignancy. Clinically, expression of ErbB2 is closely related to the prognosis of patients; highly ErbB2-expressed patients are liable to tumor metastases, with short survival. Because of a significant difference in expression of ErbB2 between normal and tumor cells, ErbB2 has been an ideal target for immunobiological cancer therapy and also an interesting molecule in the research field of tumor therapy. However, the existing CAR-NK cells feature substantial toxic and side effects and unabiding tumor cell killing effect.

### SUMMARY

In view of this, the objective of the present invention is to provide an anti-ErbB2 single chain antibody, a human ErbB2-targeting chimeric antigen receptor, a recombinant vector, a recombinant cell, and an application thereof; the anti-ErbB2 single-chain antibody can specifically recognize a tumor antigen, avoid off-target effect of CAR-NK cells, and has excellent cytotoxicitic effects to tumor cells; the NK immune cell modified by the human ErbB2-targeting chimeric antigen has a high ability to kill tumor cells continuously, reducing the risk of cytokine storm.

In order to achieve the foregoing invention objective, the present invention provides the following technical solutions.

The present invention provides an anti-ErbB2 single chain antibody, having an amino acid sequence depicted in SEQ ID No.1.

The present invention provides a gene encoding the anti-ErbB2 single chain antibody, having a nucleotide sequence depicted in SEQ ID No.2.

The present invention provides a human ErbB2-targeting chimeric antigen receptor including the single chain antibody, having an amino acid sequence depicted in SEQ ID No.3.

The present invention provides a gene encoding the chimeric antigen receptor, having a nucleotide sequence depicted in SEQ ID No.4.

The present invention provides a recombinant vector, including a gene encoding the chimeric antigen receptor and an initial vector.

Preferably, the initial vector is a lentivirus vector.

Preferably, the lentivirus vector is pCDH-MSCV-MCS-EF1-copGFP.

The present invention provides a recombinant cell, including a natural killer (NK) cell and the recombinant vector.

Preferably, the NK cell is an NK92 cell.

The present invention provides an application of the recombinant cell in the preparation of targeted drugs for cancers.

**Beneficial effects of the present invention:** The present invention is to provide an anti-ErbB2 single chain antibody, a human ErbB2-targeting chimeric antigen receptor, a recombinant vector, a recombinant cell, and an application thereof; in the present invention, the anti-ErbB2 single chain antibody is a single chain antibody targeting breast cancer cells (ScFv), which has binding activity of human breast cancer cells. In the present invention, the human ErbB2-targeting chimeric antigen receptor, in succession, includes a GM-CSF signal peptide domain, an anti-ErbB2 single chain antibody domain, a CD8 transmembrane domain, a CD28 intracellular domain, a 4-1BB intracellular domain, and a CD3ζ intracellular domain, which can specifically bind to a tumor cell surface antigen, and transfect the human ErbB2-targeting chimeric antigen receptor into a cell to acquire a recombinant cell; the human ErbB2-targeting chimeric antigen receptor can ensure the targeted localization of the recombinant cell in the tumor cell, play a killing role and avoid off-target effect of the recombinant cell, conferring a good tumor cell killing role to the recombinant cell.

The recombinant cell of the present invention has a similar affinity to a naive natural killer (NK) cell; moderate affinity helps the NK cell turn to the next target cell after the chimeric antigen receptor binding triggers killing, having an excellent killing effect to tumor cell continuously while the response to cytokine release becomes milder.. According to embodiments, for the example of HER2 antigen target, the human ErbB2-targeting chimeric antigen receptor of the present invention exhibits an everlasting and multiple tumor cell-killing capacity, while the recombinant NK cell including the human ErbB2-targeting chimeric antigen receptor has a high ability to kill tumor cells continuously, reducing the risk of cytokine storm.

### BRIEF DESCRIPTION OF THE DRAWINGS

FIG. 1 shows EC50 values of different monoclonal antibodies screened in Embodiment 1;
FIG. 2 shows histograms of analysis of antibody subtypes of the anti-ErbB2 single chain antibody described in Embodiment 1;
FIG. 3 shows expression of GFP of CAR-NK cells analyzed and separated by flow cytometry versus wild-type NK92 cells;
FIG. 4 shows ErbB2 protein binding capacities of CAR-NK cells analyzed and separated by flow cytometry versus wild-type NK92 cells;
FIG. 5 shows a comparison of 19G9 CAR-NK versus 4D5 CAR-NK cells with respect to continuous killing capacity of ErbB2-expressing target cells;
FIG. 6 shows a comparison of 19G9 CAR-NK versus 4D5 CAR-NK cells with respect to killing ability of ErbB2-expressing target cells;
FIG. 7 shows a comparison of 19G9 CAR-NK versus 4D5 CAR-NK cells with respect to contact rate of ErbB2-expressing target cells.

### DETAILED DESCRIPTION

The present invention provides an anti-ErbB2 single chain antibody, having an amino acid sequence depicted in SEQ ID No.1; the detailed amino acid sequence depicted in SEQ ID No.1 is as follows:

LEQSGPGILQPSQTLSLICSFSGFSLSTSGMGVSWIRQPSGKGLEWLAHIYWDDDKRY
NPSLKSRLTISKDTSSKQVFLKITSVDTADTATYYCALFAYYNYDGTVDYWGLGTSVTVS
SGGGGSGGGGSGGGGSDIVMTQTPSSLAVSVGEKVTMSCKSSQSLLHSRTRKNYLAWYQ
QKLGQSPKLLIYWASTRESGVPDRFTGSGSGTDFTLTISSVEAEDLAVYYCKQSYNLYTFG
GGTKLEIK.

In the present invention, the anti-ErbB2 single chain antibody includes a light chain variable region, a heavy chain variable region, and a linker sequence linking the light chain variable region with the heavy chain variable region; the amino acid sequence of the light chain variable region is depicted in SEQ ID No.5; the amino acid sequence of the heavy chain variable region is depicted in SEQ ID No.6; the amino acid sequence of the linker sequence is depicted in SEQ ID No.7.

The present invention further provides a gene encoding the anti-ErbB2 single chain antibody, having a nucleotide sequence depicted in SEQ ID No.2; the detailed nucleotide sequence depicted in SEQ ID No.2 is as follows:

ctggagcagtcaggccctgggatattgcagccctcccagaccctcagtttgatctgttctttctctgggttttcactgagtacttctggtatg
ggtgtaagctggattcgccagccttcaggaaagggtctggagtggctggcacacatttactgggatgatgacaagcgctataacccatccct
gaagagccggctcacaatctccaaggatacctccagcaagcaggttttcctcaagatcaccagtgttgacactgcagatactgccacatacta
ctgtgctctcttcgcctactataactacgacgggactgtggactactggggtctaggaacctcagtcaccgtctcctcaggcggaggcggatc
aggtggtggcggatctggaggtggcggaagcgacattgtgatgacacagactccatcctccctggctgtgtcagtaggagagaaggtcact
atgagctgcaaatccagtcagagtctgctccacagtagaacccgaaagaactatttggcttggtatcagcagaaactagggcagtctcctaaa
ctactgatctactgggcatccactagggaatctggggtccctgatcgcttcacaggcagtggctctgggacagatttcactctcaccatcagca
gtgtggaggctgaagacctggcagtttattactgcaagcaatcttataatctgtacacgttcggaggggggaccaagctggaaataaaa.

In the present invention, the gene encoding the anti-ErbB2 single chain antibody includes genes of the light chain variable region, the heavy chain variable region, and the linker sequence; the nucleotide sequence of the gene encoding the light chain variable region is depicted in SEQ ID No.8; the nucleotide sequence of the gene encoding the heavy chain variable region is depicted in SEQ ID No.9; the nucleotide sequence of the gene encoding the linker sequence is depicted in SEQ ID No.10.

The present invention provides a human ErbB2-targeting chimeric antigen receptor including the single chain antibody, having an amino acid sequence depicted in SEQ ID No.3; the detailed amino acid sequence depicted in SEQ ID No.3 is as follows:

LLLVTSLLLCELPHPAFLLIPLEQSGPGILQPSQTLSLICSFSGFSLSTSGMGVSWIRQPS
GKGLEWLAHIYWDDDKRYNPSLKSRLTISKDTSSKQVFLKITSVDTADTATYYCALFAYY
NYDGTVDYWGLGTSVTVSSGGGGSGGGGSGGGGSDIVMTQTPSSLAVSVGEKVTMSCK
SSQSLLHSRTRKNYLAWYQQKLGQSPKLLIYWASTRESGVPDRFTGSGSGTDFTLTISSVE
AEDLAVYYCKQSYNLYTFGGGTKLEIKATTTATATTTGGGCACCACTTGCAGGGACGTG
CGGCGTACTGCTTCTGTCTCTCGTGATTACTCTTTATTGTAATCATAGGAACRSKRSRLL
HSDYMNMTPRRPGPTRKHYQPYAPPRDFAAYRSRFSVVKRGRKKLLYIFKQPFMRPVQT
TQEEDGCSCRFPEEEEGGCELRVKFSRSADAPAYQQGQNQLYNELNLGRREEYDVLDKR
RGRDPEMGGKPRRKNPQEGLYNELQKDKMAEAYSEIGMKGERRRGKGHDGLYQGLSTA
TKDTYDALHMQALPPR.

In the present invention, the human ErbB2-targeting chimeric antigen receptor, in succession, includes a GM-CSF signal peptide domain, an anti-ErbB2 single chain antibody domain, a CD8 transmembrane domain, a CD28 intracellular domain, a 4-1BB intracellular domain, and a CD3ζ intracellular domain; in the present invention, the amino acid sequences of the GM-CSF signal peptide domain, the CD8 transmembrane domain, the CD28 intracellular domain, the 4-1BB intracellular domain, and the CD3ζ intracellular domain are successively depicted in SEQ ID No. 11 to SEQ ID No.15. In the present invention, the anti-ErbB2 single chain antibody domain is capable of recognizing a tumor cell antigen and targeting a tumor cell specifically; the GM-CSF signal peptide promotes transmembrane transport of CAR molecular protein after synthesis; the CD8 transmembrane domain provides a structure of transmembrane expression of CAR molecule; combination of the CD28 intracellular domain, the 4-1BB intracellular domain, and the CD3ζ intracellular domain provides a signal that activates and cytotoxtic effects after an NK cell binds to a target cell.

The present invention provides a gene encoding the chimeric antigen receptor, having a nucleotide sequence depicted in SEQ ID No.4; the detailed nucleotide sequence depicted in SEQ ID No.4 is as follows:

cttctcctggtgacaagccttctgctctgtgagttaccacacccagcattcctcctgatcccactggagcagtcaggccctgggatattg
cagccctcccagaccctcagtttgatctgttctttctctgggttttcactgagtacttctggtatgggtgtaagctggattcgccagccttcaggaa
agggtctggagtggctggcacacatttactgggatgatgacaagcgctataacccatccctgaagagccggctcacaatctccaaggatacc
tccagcaagcaggttttcctcaagatcaccagtgttgacactgcagatactgccacatactactgtgctctcttcgcctactataactacgacgg
gactgtggactactggggtctaggaacctcagtcaccgtctcctcaggcggaggcggatcaggtggtggcggatctggaggtggcggaag
cgacattgtgatgacacagactccatcctccctggctgtgtcagtaggagagaaggtcactatgagctgcaaatccagtcagagtctgctcca
cagtagaacccgaaagaactatttggcttggtatcagcagaaactagggcagtctcctaaactactgatctactgggcatccactagggaatc
tggggtccctgatcgcttcacaggcagtggctctgggacagatttcactctcaccatcagcagtgtggaggctgaagacctggcagtttatta
ctgcaagcaatcttataatctgtacacgttcggaggggggaccaagctggaaataaaattcgtgccagtattcctgcccgcgaagcccactac
tactcccgcccctaggccacctacaccggcccccacaattgcatcacaaccactcagcctccggcccgaagcttgccgccccgctgctgg
gggcgccgtgcacactcggggcctggactttgcctgcgatatttatatttgggcaccacttgcaggacgtgcggcgtactgcttctgtctctc
gtgattactctttattgtaatcataggaaccgatctaagcggtccagactgctccacagtgattacatgaacatgaccccacgcaggcctgggc
caacacgaaaacactatcagccttacgcacctccccgcgactttgctgcctaccgctctagattcagcgtggtgaagcgaggaagaaaaaa
actcctttatatattcaagcagcctttcatgcgacctgtccagacaactcaggaagaagacggctgttcttgtaggttcccagaagaggaagag
ggaggctgcgagctgcgcgtcaaattctcccgatcagcagatgcacccgcataccaacagggacagaaccagctgtacaacgaactgaat
cttggaagaagggaggagtacgatgtgcttgataaaaggcgcggccgggaccccgaaatgggcggtaagcctagacgaaaaaatcctca
agagggcctgtataatgagcttcagaaagacaagatggctgaggcgtactctgaaatagggatgaaaggagaaagaaggcggggaaaag
gccatgatggcctgtaccagggcctcagcaccgctaccaaggacacatacgatgcccttcatatgcaggccctcccccacgg.

In the present invention, the gene of the human ErbB2-targeting chimeric antigen receptor encoding the single chain antibody, in succession, includes genes encoding the GM-CSF signal peptide domain, the anti-ErbB2 single chain antibody domain, the CD8 transmembrane domain, the CD28 intracellular domain, the 4-1BB intracellular domain, and the CD3ζ intracellular domain; in the present invention, nucleotide sequences of the genes encoding the GM-CSF signal peptide domain, the CD8 transmembrane domain, the CD28 intracellular domain, the 4-1BB intracellular domain, and the CD3ζ intracellular domain are successively depicted in SEQ ID No.16 to SEQ ID No.20.

The present invention further provides a recombinant vector, including a gene encoding the chimeric antigen receptor and an initial vector. In the present invention, the initial vector is preferably a lentivirus vector, and more preferably a pCDH-MSCY-MCS-EF1-copGFP vector. In the present invention, the pCDH-MSCV-MCS-EF1-copGFP is preferably commercially available. In the present invention, the recombinant vector is preferably acquired by artificial synthesis of the gene encoding the chimeric antigen receptor, followed by subcloning into the initial vector; in the present invention, the artificial synthesis is preferably entrusted to Genewiz Co., Ltd.; the subcloning method is not particularly limited in the present invention, but conventional methods known in the art may be used. In specific embodiments of the present invention, preparation of the recombinant vector and packaging of the lentivirus vector are preferably entrusted to ABM Industries Inc.

The present invention provides a recombinant cell, including an NK cell and the recombinant vector. In the present invention, the NK cell is preferably an NK92 cell; in the present invention, the NK92 cell is preferably purchased from the cell bank of ATCC. In the present invention, the recombinant vector is transfected into the NK92 cell to acquire the recombinant cell; in the present invention, for the infection, the multiplicity of infection (MOI) is preferably (90-110):1, and more preferably 100:1; specific operations of the infection are not particularly limited in the present invention, but normal operations known in the art may be used.

The present invention further provides an application of the recombinant cell in the preparation of targeted drugs for cancers. The recombinant cell of the present invention is capable of recognizing tumor cell specifically and having excellent cytotoxictic effects to tumor cell continuously. In the present invention, the targeted drugs for cancers use the recombinant cell as the only active ingredient and use the recombinant cell and other acceptable components of the recombinant cell as active ingredients alternatively; the targeted drugs for cancers further include acceptable excipients of the recombinant cell.

The technical solution provided by the present invention will be described in detail in connection with the following embodiments, but it should not be construed as a limitation to the scope of the present invention.

### Embodiment 1

### Preparation of anti-ErbB2 single chain antibody 19G9

### S1, synthesis of ErbB2-Domain4 gene

The nucleic acid sequence of ErbB2-Domain4 gene is as follows:

atggagctggcggccttgtgccgctgggggctcctcctcgccctcttgccccccggagccgcgagcacctgccaccagctgtgcgc
ccgagggcactgctggggtccagggcccacccagtgtgtcaactgcagccagttccttcggggccaggagtgcgtggaggaatgccgag
tactgcaggggctccccagggagtatgtgaatgccaggcactgtttgccgtgccaccctgagtgtcagccccagaatggctcagtgacctgt
tttggaccggaggctgaccagtgtgtggcctgtgcccactataaggaccctcccttctgcgtggcccgctgccccagcggtgtgaaacctga
cctctcctacatgcccatctggaagtttccagatgaggagggcgcatgccagccttgccccatcaactgcacccactcctgtgtggacctgga
tgacaagggctgccccgccgagcagagagccagccctctgacgtccatcatctctgcggtggttggcattctgctggtcgtggtcttggggg
tggtctttgggatcctcatc (SEQ ID No.21).

During synthesis, restriction sites of restriction endonucleases NdeI and XhoI were added to the upstream and downstream of the ErbB2-Domain4 gene to acquire ErbB2-Domain4 inserts, respectively.

### S2, expression and purification of ErbB2-Domain4 protein

Separately, 20 µg each of vector pET-28a plasmid and ErbB2-Domain4 insert were digested with 30 IU each of restriction endonucleases NdeI and XhoI for 1 h, 50 ng each of restriction fragments were recovered, and 5000 U of T4 ligase (Neb) was added and ligated to the digested plasmid pET-28a for 1 h.

The well-ligated recombinant vector was heat shocked for 90 s and transformed into chemically competent *Escherichia coli* (*E. coli*) BL21(DE3) (purchased from Beijing TransGen Biotech Co., Ltd.). Clones were picked up for sequencing and verification, and correctly verified strains were seeded in LB medium supplemented with 50 µg/ml ampicillin to culture butterfly seed liquid overnight. One milliliter of the seed liquid was seeded into 100 mL of LB medium and cultured for 2-4 h till mid-log phase (spectrophotometrically, absorbance at 550 nm ranged from 5.0 to 1.0). Then, IPTG was added to a final concentration of 1 mmol/L, followed by induction for 2 h and then centrifugation for 15 min at 5000 ×g to collect bacterial cells; after each gram of bacterial cells were resuspended with 3 mL of STE buffer (purchased from Beijing Solarbio Science & Technology Co., Ltd.), each gram of wet *E. coli* cells were agitated with 4 µL of 0.1 mol/L PMSF and 80 µl of 10 mg/ml lysozyme for 20 min, followed by 4 mg of deoxycholic acid per gram of wet *E. coli* cells. The bacterial cells were disrupted by ultrasonic cell disruptor (VCX600, Sonics and Materials, Inc.) for 12 min. The ultrasonic mode was 45% energy at a 45 s interval every 15 working days.

After centrifugation, precipitates were collected. Inclusion bodies were oscillated and washed with STE buffer containing 0.5% Triton X100 for 15 min, and then were collected by centrifugation at 15000 ×g. The inclusion bodies were dissolved in STE buffer supplemented with 8 M urea and 0.1 mol/L PMSF, and then pH value was adjusted to 8.0 with hydrochloric acid. Protein was purified by means of Ni column affinity chromatography; ErbB2-Domain4 protein was collected as antigen for use. The amino acid sequence of the ErbB2-Domain4 protein is as follows:

MELAALCRWGLLLALLPPGAASTCHQLCARGHCWGPGPTQCVNCSQFLRGQECVE
ECRVLQGLPREYVNARHCLPCHPECQPQNGSVTCFGPEADQCVACAHYKDPPFCVARCP
SGVKPDLSYMPIWKFPDEEGACQPCPINCTHSCVDLDDKGCPAEQRASPLTSIISAVVGILL
VVVLGVVFGILI (SEQ ID No.22).

### S3, immunization of mice:

Using the purified ErbB2-Domain4 protein as antigen, three female BALB/c mice aged 6-8 weeks were immunized thrice. The interval between the first two immunizations was two weeks; the dose was 12.5 µg/mouse; tail blood was sampled at 10 days after the second immunization. The last recombinant antigen injection was given via the tail vein at 3 days prior to fusion; the dose was 60 µg/mouse; mice were boosted once.

### S4, fusion of myeloma cells with immune spleen cells:

Mouse myeloma SP2/0 cells (purchased from Cell Resource Center, Institute of Basic Medical Sciences of the Chinese Academy of Medical Sciences) were cultured in 10% FBS supplemented DMEM and subcultured at a 1:3 vaccination ratio every 24 h for use. Immune mouse spleens were collected, ground, and passed through a cell mesh to acquire mouse spleen cells. After mouse spleen cells and SP2/0 cells were washed with DMEM thrice, the ratio of spleen cell to SP2/0 cell was adjusted to 1:10; after reaction with 50% PEG1500 for 1-2 min, cells were diluted with HAT supplemented complete medium, seeded in a 96-well plate, and cultured at 37°C in a 5% CO₂ atmosphere.

### S5, screening and cloning of positive hybridoma cells:

Cell wells which were determined as positive by means of indirect ELISA in duplicate consecutively were subcloned by means of limiting dilution. In case of specific anti-ErbB2 antibodies determined in cell wells, wells of hybridoma cells with high antibody titer, monoclonal growth and good morphology were selected to sequentially reclone in the same manner, at least two times; the hybridoma cells in the cloned positive wells were transferred into a 24-well plate; till the hybridoma cells therein grew well, they could be cryopreserved.

### S6, production of monoclonal antibody:

The established hybridoma cells were seeded in 10% fetal bovine serum (FBS) supplemented DMEM at 10e6 cells/ml for scale-up culture; cells were centrifuged every 48 h and seeded in a fresh medium at 10e6 cells/ml again after supernatant was discarded. After acquisition of a sufficient amount of cells, the original medium was removed by centrifugation; then, the cells were seeded in serum-free DMEM at 10e6 cells/ml, cultured for 72 h, and centrifuged, and the supernatant was collected.

### S7, purification of monoclonal antibody:

The cell culture supernatant where cells were removed by centrifugation was purified by means of HiTrap protein G affinity chromatography, and therapeutic mouse anti-human ErbB2 monoclonal antibody 19G9 was acquired.

### Titration of anti-ErbB2 monoclonal antibody 19G9

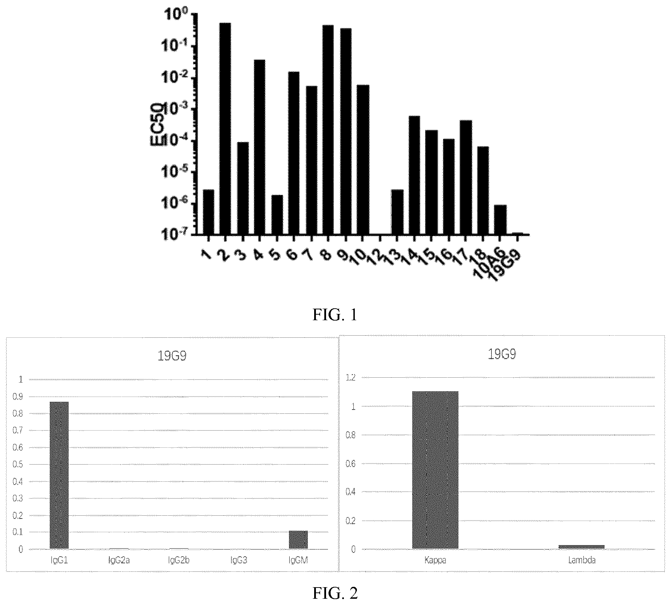

Gradient dilution was conducted on the anti-ErbB2 monoclonal antibody 19G9, and titration was conducted by means of indirect ELISA. The method for titrating the monoclonal antibody by means of indirect ELISA was to: coat the ErbB2-Domain4 protein at 100 ng/well, dilute the anti-ErbB2 monoclonal antibody 19G9 at 1:10000, 1:20000, 1:40000, 1:80000, 1:160000, 1:320000, 1:640000, 1:1280000, 1:2560000, 1:5120000, 1:10240000, and 1:20480000, respectively, and measure their absorbance at 450 nm by means of indirect ELISA. The titer was the maximum dilution of the anti-ErbB2 monoclonal antibody 19G9 reacting with target antigen of immune protein, and the ratio of well reading to negative control value being >2.1 was determined as positive. Also, EC50 values were calculated. Results are shown in FIG. 1. The EC50 value of the anti-ErbB2 monoclonal antibody 19G9 is 10⁻⁷ mol/L.

For the anti-ErbB2 monoclonal antibody 19G9, Ig subclasses of monoclonal antibodies of anti-human ErbB2 protein were identified using Ig class and subclass ELISA kits (Sino Biological Inc.). Results are shown in FIG. 2. 19G9 was determined as a mouse IgG1-Kappa antibody.

### Preparation of anti-ErbB2 monoclonal antibody 19G9

The sequences of the heavy and light chain variable regions of the anti-ErbB2 monoclonal antibody 19G9 and the linker sequence linking the heavy chain variable region with the light chain variable region constituted the anti-ErbB2 monoclonal antibody 19G9.

The preparation method of the anti-ErbB2 monoclonal antibody 19G9 is as follows:
Using TRIZOL reagent (Thermo Fisher), total RNA was extracted from 19G9 hybridoma cells, specifically as follows: homogenizing 10⁶ cells with TRIZOL reagent, then adding chloroform, centrifuging for 10 min at 12000 ×g at 2-8°C, and adding 0.5 mL of isopropanol; high-speed freezing and centrifuging for 10 min at 12000 ×g. RNA precipitates were washed with 75% ethanol once and dried, and RNAs were dissolved in RNase-free water. After concentration determination, 1 µg of cDNA was prepared by Superscript III Reverse Transcriptase Kit. Sequences of light and heavy chains were prepared by PCR method. The PCR system was 50 µl, including: 100 ng of cDNA, 2.5 µL each of 10 nM forward and reverse primers, 25µL of Taq 2X Mastermix(ABM), and finally diluting to a volume of 50 µL with water. The analyte was predenaturated for 3 min at 94°C, denaturated for 30s at 94°C, annealed at 60°C for 0.5 min, and elongated at 72°C for 1 min, for 35 cycles. The PCR product was submitted to Genewiz Co., Ltd. for Sanger sequencing, and sequences of the heavy and light chain variable regions were acquired.

PCR primers of the light and heavy chains are as follows:
Reverse primer of the heavy chain: ggaagatctatagacagatgggggtgtcgttttggc (SEQ ID No.23)
Forward primer of the heavy chain: cttccggaattcgaggtcaagctgcagcagtcagg (SEQ ID No.24)
Reverse primer of the light chain: ggtgcatgcggatacagttggtgcagcatc (SEQ ID No.25)
Forward primer of the light chain: gggagctcgacattgtgctgacacaatctcct (SEQ ID No.26)

The sequence of the heavy chain variable region, the linker sequence, and the sequence of the light chain variable region were ligated in succession to acquire the gene of the anti-ErbB2 monoclonal antibody 19G9, specifically as follows (SEQ ID No.2):

ctggagcagtcaggccctgggatattgcagccctcccagaccctcagtttgatctgttctttctctgggttttcactgagtacttctggtatg
ggtgtaagctggattcgccagccttcaggaaagggtctggagtggctggcacacatttactgggatgatgacaagcgctataacccatccct
gaagagccggctcacaatctccaaggatacctccagcaagcaggttttcctcaagatcaccagtgttgacactgcagatactgccacatacta
ctgtgctctcttcgcctactataactacgacgggactgtggactactggggtctaggaacctcagtcaccgtctcctcaggcggaggcggatc
aggtggtggcggatctggaggtggcggaagcgacattgtgatgacacagactccatcctccctggctgtgtcagtaggagagaaggtcact
atgagctgcaaatccagtcagagtctgctccacagtagaacccgaaagaactatttggcttggtatcagcagaaactagggcagtctcctaaa
ctactgatctactgggcatccactagggaatctggggtccctgatcgcttcacaggcagtggctctgggacagatttcactctcaccatcagca
gtgtggaggctgaagacctggcagtttattactgcaagcaatcttataatctgtacacgttcggaggggggaccaagctggaaataaaa.

The amino acid sequence of the translated anti-ErbB2 monoclonal antibody 19G9 is as follows (SEQ ID No.1):

LEQSGPGILQPSQTLSLICSFSGFSLSTSGMGVSWIRQPSGKGLEWLAHIYWDDDKRY
NPSLKSRLTISKDTSSKQVFLKITSVDTADTATYYCALFAYYNYDGTVDYWGLGTSVTVS
SGGGGSGGGGSGGGGSDIVMTQTPSSLAVSVGEKVTMSCKSSQSLLHSRTRKNYLAWYQ
QKLGQSPKLLIYWASTRESGVPDRFTGSGSGTDFTLTISSVEAEDLAVYYCKQSYNLYTFG
GGTKLEIK.

### Embodiment 2

### Preparation of recombinant vector

The expressed sequence was arranged in the GM-CSF signal peptide-19G9 ScFv-CD8 TM-41BBcyto-CD28cyto-CD3ζ manner, in which the sequence of each fragment is as follows:
GM-CSF signal peptide (SEQ ID No.16)
   cttctcctggtgacaagccttctgctctgtgagttaccacacccagcattcctcctgatccca
19G9 single chain antibody domain (SEQ ID No.2) 
ctggagcagtcaggccctgggatattgcagccctcccagaccctcagtttgatctgttctttctctgggttttcactgagtacttctggtatg
ggtgtaagctggattcgccagccttcaggaaagggtctggagtggctggcacacatttactgggatgatgacaagcgctataacccatccct
 
gaagagccggctcacaatctccaaggatacctccagcaagcaggttttcctcaagatcaccagtgttgacactgcagatactgccacatacta
ctgtgctctcttcgcctactataactacgacgggactgtggactactggggtctaggaacctcagtcaccgtctcctcaggcggaggcggatc
aggtggtggcggatctggaggtggcggaagcgacattgtgatgacacagactccatcctccctggctgtgtcagtaggagagaaggtcact
atgagctgcaaatccagtcagagtctgctccacagtagaacccgaaagaactatttggcttggtatcagcagaaactagggcagtctcctaaa
ctactgatctactgggcatccactagggaatctggggtccctgatcgcttcacaggcagtggctctgggacagatttcactctcaccatcagca
gtgtggaggctgaagacctggcagtttattactgcaagcaatcttataatctgtacacgttcggaggggggaccaagctggaaataaaa
CD8 transmembrane domain (SEQ ID No.17) 
ttcgtgccagtattcctgcccgcgaagcccactactactcccgcccctaggccacctacaccggcccccacaattgcatcacaaccac
tcagcctccggcccgaagcttgccgccccgctgctgggggcgccgtgcacactcggggcctggactttgcctgcgatatttatatttgggca
ccacttgcagggacgtgcggcgtactgcttctgtctctcgtgattactctttattgtaatcataggaac
CD28 intracellular domain (SEQ ID No.18) 
cgatctaagcggtccagactgctccacagtgattacatgaacatgaccccacgcaggcctgggccaacacgaaaacactatcagcct
tacgcacctccccgcgactttgctgcctaccgctct
 4-1BB intracellular domain (SEQ ID No.19) 
agattcagcgtggtgaagcgaggaagaaaaaactcctttatatattcaagcagcctttcatgcgacctgtccagacaactcaggaag
aagacggctgttcttgtaggttcccagaagaggaagagggaggctgcgagctg
 CD3ζ intracellular domain (SEQ ID No.20)

cgcgtcaaattctcccgatcagcagatgcacccgcataccaacagggacagaaccagctgtacaacgaactgaatcttggaagaag
ggaggagtacgatgtgcttgataaaaggcgcggccgggaccccgaaatgggcggtaagcctagacgaaaaaatcctcaagagggcctgt
ataatgagcttcagaaagacaagatggctgaggcgtactctgaaatagggatgaaaggagaaagaaggcggggaaaaggccatgatggc
ctgtaccagggcctcagcaccgctaccaaggacacatacgatgcccttcatatgcaggccctcccccccacgg

The foregoing sequences were synthesized by Genewiz Co., Ltd., subcloned into pCDH-MSCV-MCS-EF1-copGFP lentiviral plasmids, and entrusted to ABM Industries Inc. for viral packaging to acquire the recombinant vector.

### Embodiment 3

### Preparation of recombinant cell

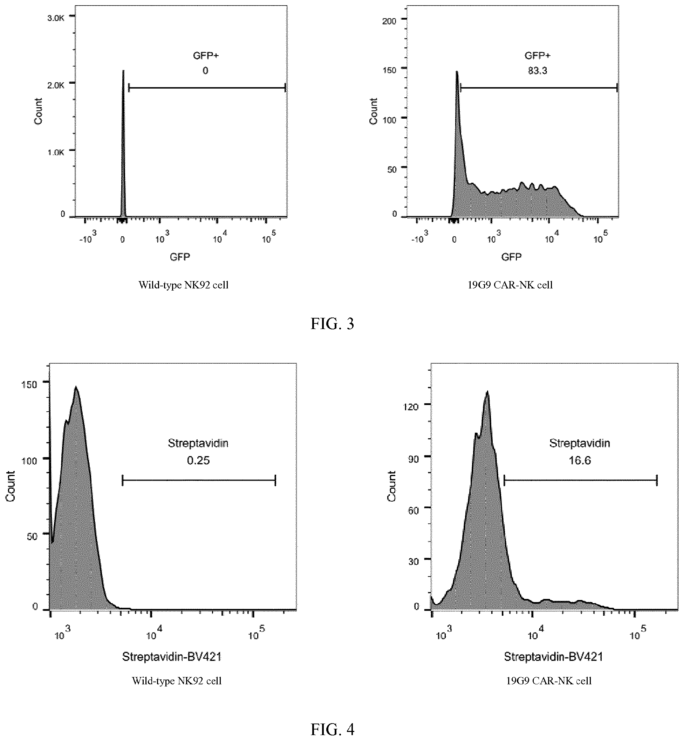

The viruses packaged in the Embodiment 2 were transfected into NK92 cells (purchased from ATCC) at an MOI (multiplicity of infection) of 100:1. The Scale-up culture was conducted on transfected NK92 cells in LY09 medium; after culture for 72 h, copGFP reporter gene (green fluorescence) positive cells were separated by flow cytometry. At the moment, the recombinant cell 19G9 CAR-NK was acquired. Results of expression of GFP of 19G9 CAR-NK cells analyzed and separated by flow cytometry versus wild-type NK92 cells are shown in FIG. 3: expression of GFP of 19G9 CAR-NK cells is high, while that of wild-type NK92 cells is zero.

Flow cytometry was used to analyze the specific binding of ErbB2 protein to 19G9 CAR-NK cell. One microgram of ErbB2-biotin(Acro) protein was co-incubated with 1000000 19G9 CAR-NK cells and NK92 cells for 30 min, respectively, and then re-incubated with 50 ng of Pacific Blue conjugated streptavidin for 15 min, followed by flow cytometry. As shown in FIG. 4, ErbB2 protein has a high affinity for 19G9 CAR-NK cells, and weakly binds to wild-type NK92 cells.

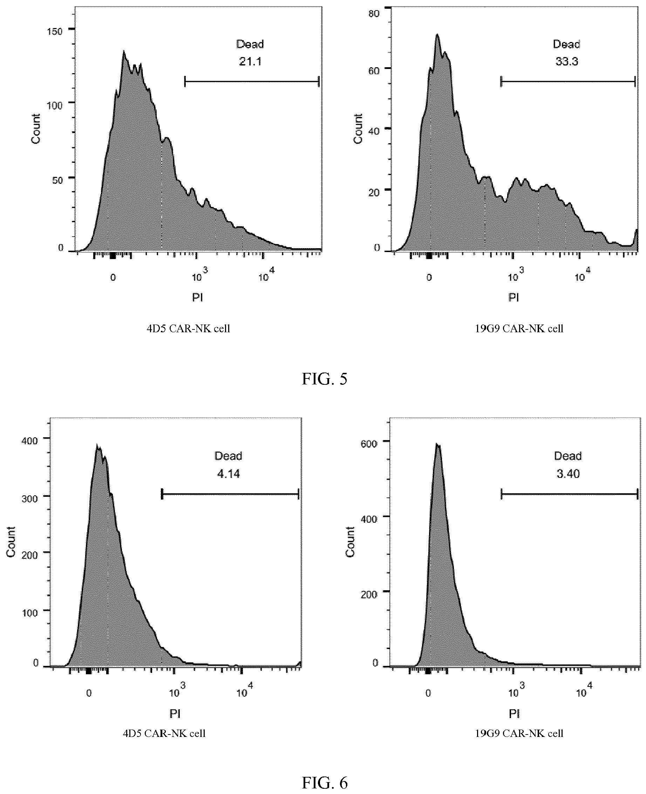

Flow cytometry was used to analyze the specific killing of 19G9 CAR-NK cells on ErbB2-expressing target cells. The cells were cultured overnight in a 24-well plate with RPMI1640 medium supplemented with 1 µg/mL of hoechst33342 and 10% FBS at 100000 target cells per well, co-incubated with 500000 19G9 CAR-NK cells and NK92 cells for 4 h, respectively, and then re-incubated with 10 µL of PI staining solution (BD) for 15 min, followed by flow cytometry. Cells with hoechst33342 fluorescence were circled, and PI-positive cell ratio was analyzed. As shown in FIG. 5, 19G9 CAR-NK cells have a high killing ability of target cells (PI-positive) and kill wild-type NK92 cells weakly.

After the sequence of the 19G9 single chain antibody domain in the plasmid vector was replaced with a 4D5ScFv sequence, the expressed sequence was arranged in the GM-CSF signal peptide-19G9 ScFv-CD8 TM-41BBcyto-CD28cyto-CD3ζ manner. Then, it was submitted to Genewiz Co., Ltd. for complete sequence synthesis, subcloned into pCDH-MSCV-MCS-EF1-copGFP lentiviral plasmids, and entrusted to ABM Industries Inc. for viral packaging. The packaged viruses were transfected into NK92 cells (from ATCC) at an MOI of 100:1. Scale-up culture was conducted on the transfected NK92 cells in LY09 medium; after culture for 72 h, copGFP reporter gene (green fluorescence) positive cells were separated by flow cytometry. At the moment, 4D5 CAR-NK cells were acquired.

An analysis was conducted on continuous killing effects of different CAR-NK cells (19G9 CAR-NK versus 4D5 CAR-NK cells) on ErbB2-expressing target cells. The cells were cultured overnight in a 24-well plate with RPMI1640 medium supplemented with 1 µg/mL of hoechst33342 and 10% FBS at 100000 target cells per well, co-incubated with 500000 19G9 CAR-NK cells and 4D5 CAR-NK for 4 h, respectively, and then re-incubated with 10 µL of PI staining solution (BD) for 15 min, followed by flow cytometry. Cells with hoechst33342 fluorescence were circled, and PI-positive cell ratio (death rate) was analyzed. Results are shown in FIG. 6, in which 19G9 CAR-NK cells have a high killing ability of target cells (PI-positive), higher than that of 4D5 CAR-NK cells. A further analysis was conducted on the death ratio (death rate) of GFP-positive effector cell PI-positive cell, and it followed that 19G9 CAR-NK cells had a low death rate, which could continuously kill target cells.

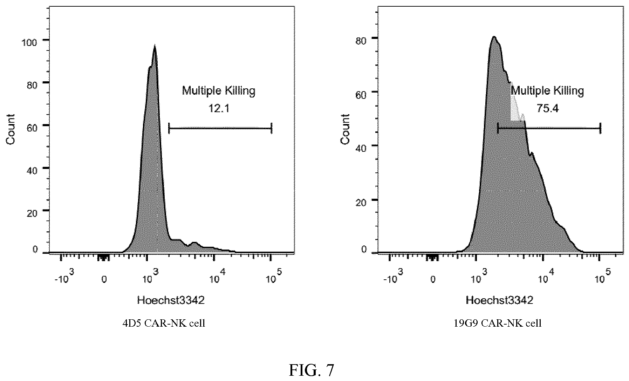

A further analysis was conducted on the ratio of hoechst33342 cells acquired by GFP-positive effector cells (contact rate of target cell). Results are shown in FIG. 7. It follows that 19G9 CAR-NK cells are mostly hoechst33342-positive and bind to more cells.

In conclusion, the anti-ErbB2 single chain antibody of the present invention can specifically recognize a tumor antigen; the NK immune cell modified by the human ErbB2-targeting chimeric antigen receptor is capable of having a higher cytotoxixity to tumor cell continuously and reducing the risk of cytokine storm.The foregoing descriptions are only preferred implementation manners of the present invention. It should be noted that for a person of ordinary skill in the art, several improvements and modifications may further be made without departing from the principle of the present invention. These improvements and modifications should also be deemed as falling within the protection scope of the present invention.

## Claims

1. An anti-ErbB2 single chain antibody, having an amino acid sequence depicted in SEQ ID No.1.

2. A gene encoding the anti-ErbB2 single chain antibody according to claim 1, having a nucleotide sequence depicted in SEQ ID No.2.

3. A human ErbB2-targeting chimeric antigen receptor comprising the single chain antibody according to claim 1, having an amino acid sequence depicted in SEQ ID No.3.

4. A gene encoding the chimeric antigen receptor according to claim 3, having a nucleotide sequence depicted in SEQ ID No.4.

5. A recombinant vector, comprising a gene encoding the chimeric antigen receptor according to claim 3 and an initial vector.

6. The recombinant vector according to claim 5, wherein the initial vector is a lentivirus vector.

7. The recombinant vector according to claim 6, wherein the lentivirus vector is pCDH-MSCV-MCS-EF1-copGFP.

8. A recombinant cell, comprising a natural killer (NK) cell and the recombinant vector according to any one of claims 5 to 7.

9. The recombinant cell according to claim 8, wherein the NK cell is an NK92 cell.

10. An application of the recombinant cell according to claim 8 or 9 in the preparation of targeted drugs for cancers.
